# NEW EUROPEAN PATENT SPECIFICATION

(11) **EP 0 091 787 B2**
(45) Date of publication and mention of the opposition decision: **09.12.1998**
(45) Mention of the grant of the patent: 04.09.1985
(21) Application number: 83301951.6
(22) Date of filing: 07.04.1983
(51) Int. Cl.: C07K 1/14, A23L 1/236, B01D 9/02

(54) **Process for crystallizing alpha-L-aspartyl-L-phenylalanine-methyl ester**
Verfahren zum Kristallisieren von alpha-L-Aspartyl-L-phenylalanin-methylester
Procédé pour cristalliser l'ester méthylique de l'alpha-L-aspartyl-L-phénylalanine

(30) Priority: 12.04.1982 JP 60671/82
(43) Date of publication of application: 19.10.1983
(73) Proprietor: AJINOMOTO CO., INC., Tokyo 104 (JP)
(72) Inventor: Naruse, Masayoshi, Yokohama-shi Kanagawa-ken (JP); Kawasaki, Haruo, Shibuya-ku Tokyo (JP); Kishimoto, Shinichi, Yokkaichi-shi Mei-ken (JP); Oura, Harutoshi, Kawasaki-shi Kanagawa-ken (JP); Nakamura, Masao, Yokohama-shi Kanagawa-ken (JP); Takeda, Hideo, Inagi-shi Tokyo (JP)
(74) Representative: Bond, Bentley George

(56) References cited:
- EP-A- 0 035 047
- DE-A- 605 121
- DE-A- 3 013 701
- US-A- 3 798 207

## Description

This invention relates to a process for crystallizing and separating L-α-aspartyl-L-phenylalanine methyl ester under specific crystallization conditions.

L-α-aspartyl-L-phenylalanine methyl ester (hereinafter abbreviated to APM) is a substance which is expected to find wide application as a new low-calorie sweetener due to its good sweetening properties.

As processes for industrially producing APM, the following processes are typical. Thus, there are known a process for binding an N-substituted aspartic acid anhydride to phenylalanine methyl ester in an organic solvent (US Patent 3,786,039), a process of directly binding a strong acid addition salt of aspartic acid anhydride to phenylalanine methyl ester (Japanese Patent Publication No. 14217/74), and a process of condensing an N-substituted aspartic acid with phenylalanine methyl ester in the presence of an enzyme and eliminating the substituent (Japanese patent Publication No. 135595/80).

In industrial production, the crystallization step for isolating APM from a reaction solution is necessary for obtaining a final product, in the processes described above. This crystallizing step is usually conducted, for example, by re-dissolving a crude product in water, organic solvent or aqueous organic solvent, by cooling the solution by heat exchange with a refrigeration medium (forced cyclization type indirect cooling system) or evaporating part of the solvent under reduced pressure (self-evaporating system) using a crystallizer equipped with a stirring means, and by dewatering and filtering out the thus precipitated crystals by means of a centrifugal separator or the like.

However, the thus obtained APM crystals are fine needle-like crystals, and therefore have extremely bad solid-liquid separability in the filtration and dewatering procedure, whereby the above-described processes have practical problems.

To illustrate one case, when 600 litres of a slurry containing APM crystals obtained by one of the above-described processes (see the Comparative Example below) is subjected to solid-liquid separation by filtering for 2 hours and dehydrating for one hour in a centrifugal separator having a diameter of 36 inches (91.4 cm) and a volume of 92 litres (number of revolutions = 1100 r.p.m.; centrifuging effect=600 G), the resulting cake has a water content of 45 to 50% or more. The term water content as used herein is defined as (amount of water in the cake/total amount of wet cake) × 100%.

In addition, there is another defect in that, when the procedures of scraping this cake and conducting solid-liquid separation of a fresh slurry containing APM crystals are repeated, the base layer of cake becomes so tightly hardened that its removal requires much labour and time.

Further, in the drying step following the crystallizing step, the drying load is too high due to the high water content of the cake, and the resulting powder has such a large bulk specific volume that it is extremely difficult to handle.

Table 1 shows powder properties of APM crystals obtained by a preferred crystallizing process of the present invention (see Example 1 below) and of crystals obtained by one conventional process (see the Comparative Example below).

**Table 1**

| | Conventional Process | Process of the Invention |
|---|---|---|
| Static specific volume (cc/g) | 6 - 7 | 3 - 4 |
| Close specific volume (cc/g) | 3 - 4 | 2 - 3 |
| Rate of dissolution (min.) | 14 - 17 | 5 - 6 |

In crystallizing other materials, it is known that the above-described problems in the crystallizing procedure can be overcome by employing a low concentration and a slow cooling rate to obtain crystals having a large diameter. With APM crystals, however, such a crystallization procedure results in the formation of needle-like crystals growing only in a longitudinal direction, thus failing to provide the expected effects. For example, when seed crystals are added to a 0.8 wt % APM solution and the solution temperature is decreased from 15°C to 5°C in two days, crystals grow 214% in length but only 15% in diameter.

According to the present invention there is provided a process for crystallizing APM on an industrial scale from its aqueous solution by cooling in an industrial crystallizer, which comprises adjusting the initial concentration of the ester so that the amount of precipitated solid phase formed after cooling is about 10 g or more per litre of solvent, cooling the solution by conductive heat transfer to form an apparently sherbet-like pseudo solid phase without effecting forced flow (that is to say without mechanical stirring or the like), and, if necessary, further cooling the system after formation of the pseudo solid phase, converting said pseudo solid phase to a slurry, subjecting the slurry to a solid-liquid separation, and drying the crystals of APM; wherein said sherbet-like pseudo solid phase comprises bundle-like crystal aggregates of APM and the solvent, has no fluidity, and may be converted into a slurry by stirring.

As a result of intensive investigations to improve the workability of the aforesaid step in the production of APM by examining various conditions, the inventors have found the following novel facts. Thus, surprisingly, it has been found that, in crystallizing APM from its solution of a certain concentration or above by cooling without stirring, the APM crystals take up the solvent into the space formed among them, and the whole solution thus appears apparently solidified, and that the crystals obtained in this state have extremely good properties in a subsequent solid-liquid separation procedure. Observation of the crystals under a scanning type electromicroscope has revealed that several needle-like crystals are bundled together to form apparently one crystal (to be described hereinafter).

The bundle-like crystal aggregates of the present invention are extremely resistant to physical impact as long as they are not under growing in a supersaturated solution, and have a 5-fold to 10-fold or more diameter as compared to conventional crystals even after being transported, separated or dried.

More surprisingly, even under such crystallizing conditions that, with ordinary substances, crystals adhere onto a heat-transferring surface to result in the formation of scale which is difficult to remove, the precipitation of APM crystals in accordance with the present invention is found to enable one to completely remove the crystal layer from the cooling surface.

As a result of intensive investigations to apply the above-described findings to an actual process, the inventors have achieved an improvement in workability in the step of precipitating APM crystals from an APM solution by cooling the solution under conditions such as to form a pseudo solid phase to obtain crystals having good separability, and have thus completed a novel crystallizing process of industrially great economical effect. As a result of further investigations, the inventors have found that, once the solution becomes a pseudo solid phase, it can maintain its good separability even when subjected to a desupersaturation procedure of rapid cooling accompanied by forced flow, which serves to increase the efficiency of the process and improve the crystallization yield.

A characteristic aspect of the present invention is that one obtains bundled large-diameter APM crystal aggregates by cooling as fast as possible an APM solution, employing such crystallizing conditions or crystallizers that natural heat transfer by convection is realized only in the very early stage of the crystallizing, heat transfer then being controlled by conduction.

According to the present invention, the solid-liquid separability and powder properties of the product can be improved, and the workability in each step can be remarkably improved. Therefore, the present invention provides an APM-crystallizing process which is economically quite advantageous. Additionally, due to the above-described properties of the product, APM crystals having a poor crystallization behaviour can be converted to APM crystals having a good crystallization behaviour by the recrystallization process in accordance with the present invention, and APM containing impurities such a diketopiperazine (DKP), a cyclized product of APM, and L-α-aspartyl-L-phenylalanine can be freed of the impurities by subjecting the impurity-containing APM to the crystallizing process of the present invention coupled with a decrease in the amount of adhering mother liquor in a solid-liquid separation and an improvement in cake washability.

The present invention will now be described, by way of example, with reference to the drawings, in which :
Fig. 1A and Fig. 1B are electron micrographs of APM crystals obtained by a process of the present invention ;
Fig. 2A and Fig. 2B are electron micrographs of APM crystals obtained by a conventional process;
Fig. 3A and Fig. 3B are electron micrographs of APM crystals obtained without causing forced flow and under such conditions that no sherbet-like phase is formed;
Figs. 4, 5, 6 and 7 show examples of crystallizers for use in the present invention;
Fig. 8 shows the solubility of APM in water;
Fig. 9 shows the crystallizer used in Example 1;
Fig. 10 shows a crystallizer used in a conventional process;
Fig. 11A and Fig. 11B show the results of comparing the process of the present invention with a conventional process with respect to the filtration rate and the dehydration rate of APM slurry; and
Fig. 12 shows the crystallizer used in Example 2.

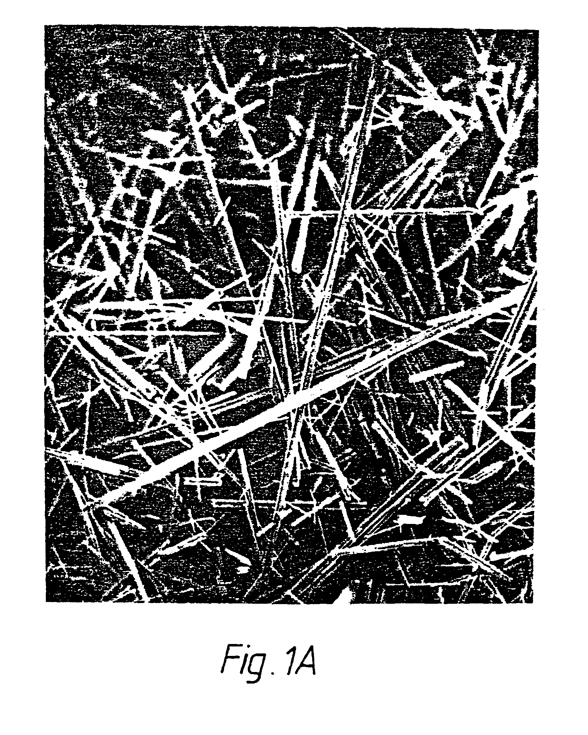

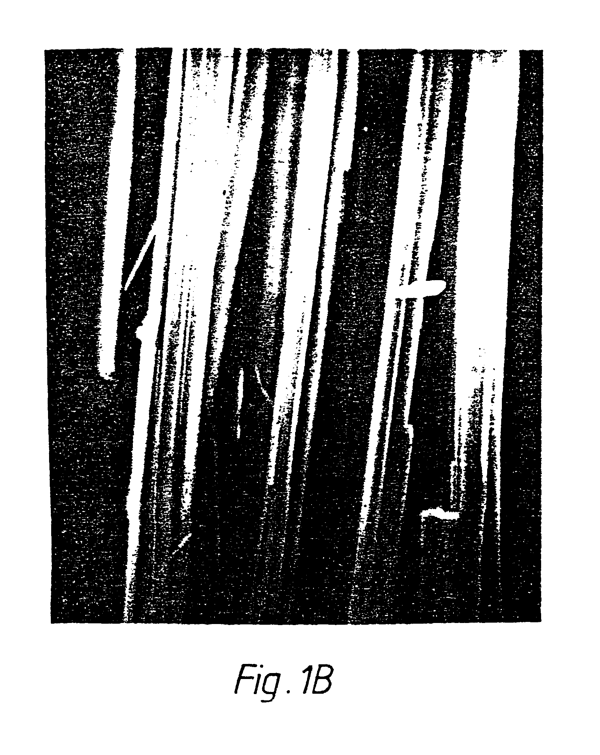

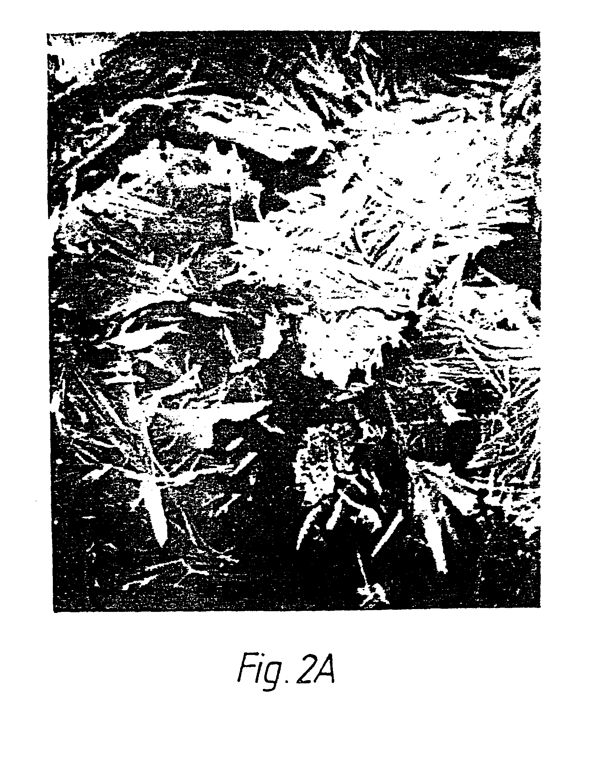

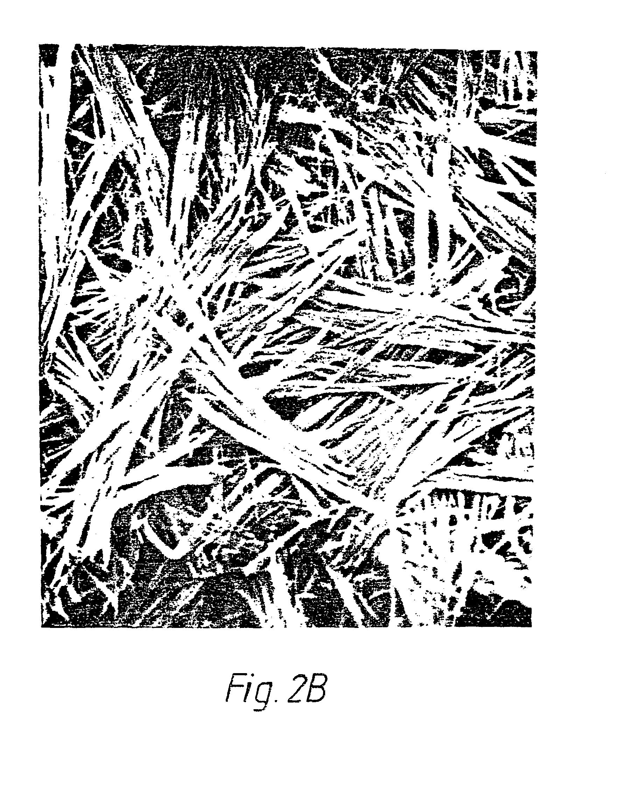

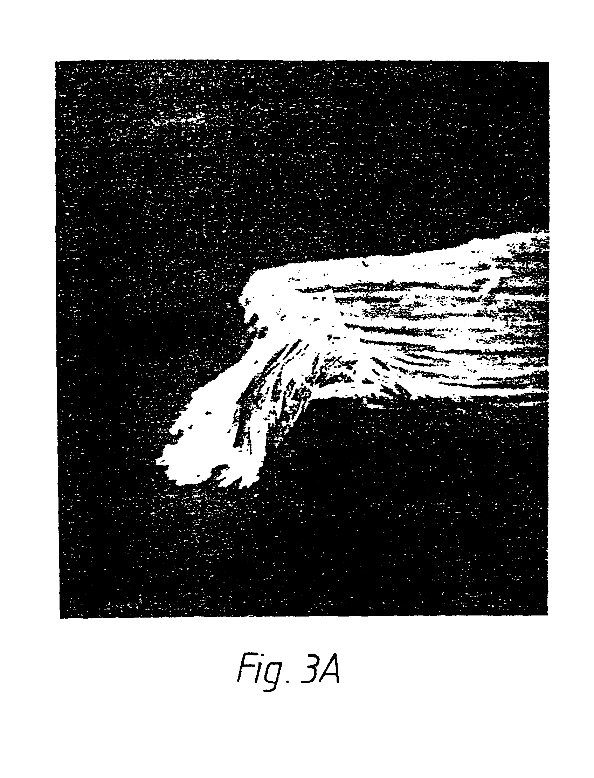

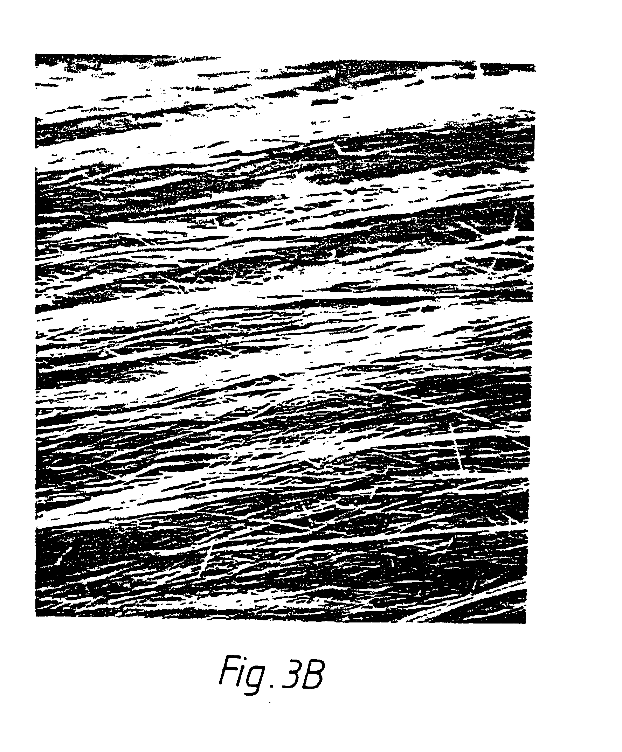

In the process of the present invention, cooling is conducted without forced flow caused, for example, by mechanical stirring. Additionally, it is desirable to render the whole solution into a sherbet-like pseudo solid phase to prevent natural flow phenomenon resulting from temperature distribution as fast as possible. For the purpose of comparison, the drawings show electron micrographs of bundled crystals obtained by the process of the present invention (Fig. 1A (×58) and Fig. 1B (× 580) ), fine crystals obtained by indirect cooling with forced flow by one of the conventional processes (Fig. 2A (× 560) and Fig. 2B (× 1280)), and dendrite crystals obtained without effecting forced flow and under such conditions that no sherbet is formed (Fig. 3A (× 51) and Fig. 3B (× 350)). From these photographs, it can be easily understood that the three crystals, which show the same results in X-ray powder diffractiometry, are clearly different from each other in shape and size due to the difference in the manner of crystallizing.

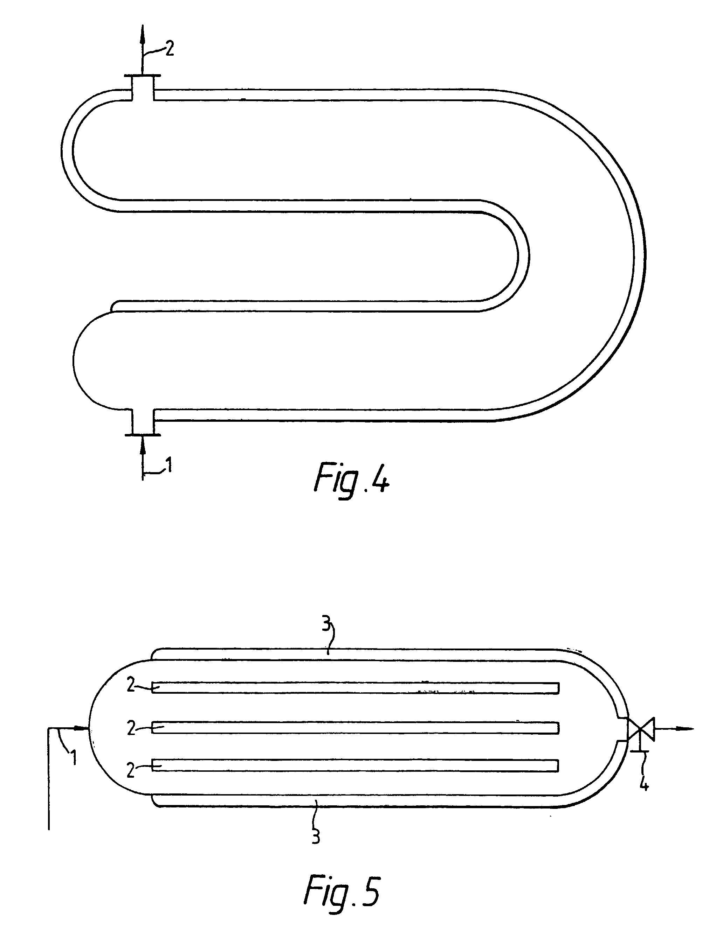

As a crystallizer for satisfying the above-described procedure at conditions, Fig. 4 shows an example of continuous crystallizers, in which a jacketed U-tube having nozzles at both ends is used. Before initiation of the crystallizing procedure, a feed solution is charged in the tube, before the initiation of cooling. At a stage where crystallization has proceeded in the tube, a feed solution is pressed into the tube through feed inlet 1 at a slow rate, upon which a sherbet-like slurry is driven out of the tube through the outlet 2. The sherbet-like slurry can be continuously obtained by cooling through heat transfer by conduction and by feeding the solution at such flow rate that the residence time is sufficient for crystallization to be completed.

Additionally, the crystallizer need not necessarily be a U-tube: any vertical or horizontal straight tube and any curved tube that does not suffer a pressure loss more than is necessary may be employed as well.

Fig. 5 shows an example of a batchwise crystallizer. A feed solution is introduced through feed inlet 1. After completion of charging of the solution, a refrigeration medium is introduced into cooling plates 2, or a cooling tube, and into jacket 3 to cool the contents. After a predetermined period of time, the discharge valve 4 is opened to discharge a sherbet-like slurry.

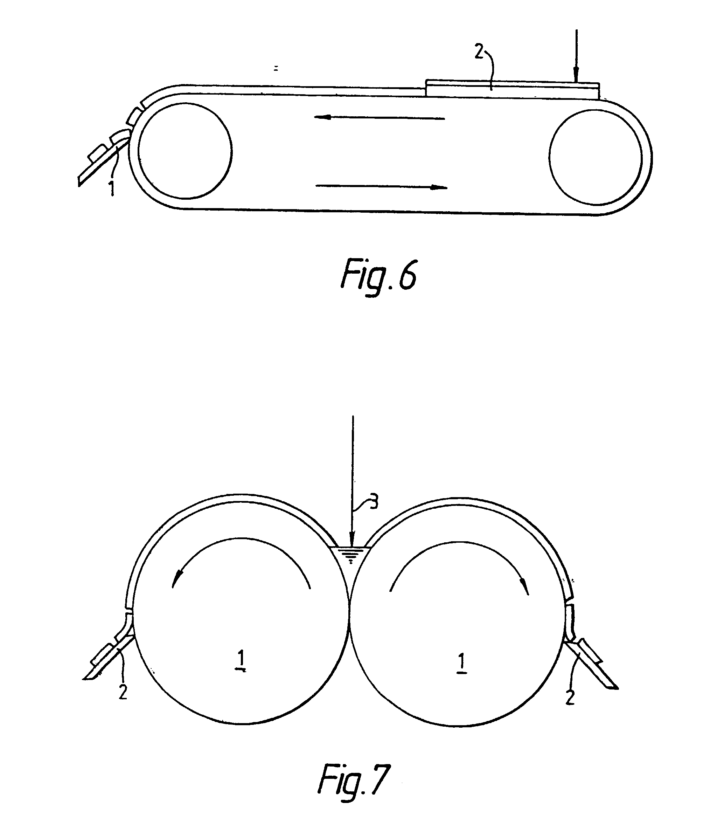

Figs. 6 and 7 show examples of the manner of conducting the process of the present invention, using a conventional apparatus. The procedures are continuous in both cases.

In Fig. 6, a rotating steel belt is used as a cooling surface (the belt being cooled, for example, by blowing a refrigeration medium to the back of the belt), and a feed solution is continuously introduced onto the belt to crystallize. The resulting sherbet-like slurry is recovered by scraping with a scraper 1 provided on the other end. In this embodiment, for the purpose of forming a thick sherbet layer on the belt, guides 2 may be provided on the sides of the belt, or a frame may fixed on the belt to thereby prevent the solution from flowing over before solidifying. In some cases, a semi-continuous procedure may be employed.

Fig. 7 shows the use of an evaporater-condenser. A feed solution is introduced into the centre 3 of two contacting drums 1 which rotate outward. The drums are cooled from inside with a refrigeration medium instead of being heated from inside with steam, and sherbet deposits on the drums as a result of crystallization. The resulting sherbet is removed by scraper 2.

These embodiments are particularly designed or intended for satisfying the aforesaid special conditions of the crystallization to be employed in the process of the present invention. The inventors are not aware that the above-described apparatuses have been used for crystallizing APM as well as other materials through heat transfer by conduction. Processes for crystallizing APM with the use of any other apparatuses which satisy the specific crystallization conditions of this invention are of course within the scope of this invention.

To attain an apparantly solidified state, the solution must contain about 10 g or more of solids per litre of the solvent. That is, in an aqueous solution system, a satisfactory recovery of APM can be attained by cooling the system to 5° C, taking the solubility of APM into consideration. Theorectically, an initial concentration of the AMP solution before crystallization of 1.5 wt % suffices since the saturation concentration at this temperature is 0.5%.

However, in a low supersaturated region, the crystallizing rate is too slow. Therefore, the aqueous system must in practice contain about 2 wt % or more APM for forming the sherbet state. In order to obtain crystals having a large diameter, the solidification must proceed at faster rate. For this purpose, the initial concentration is desirably about 3 wt % or more.

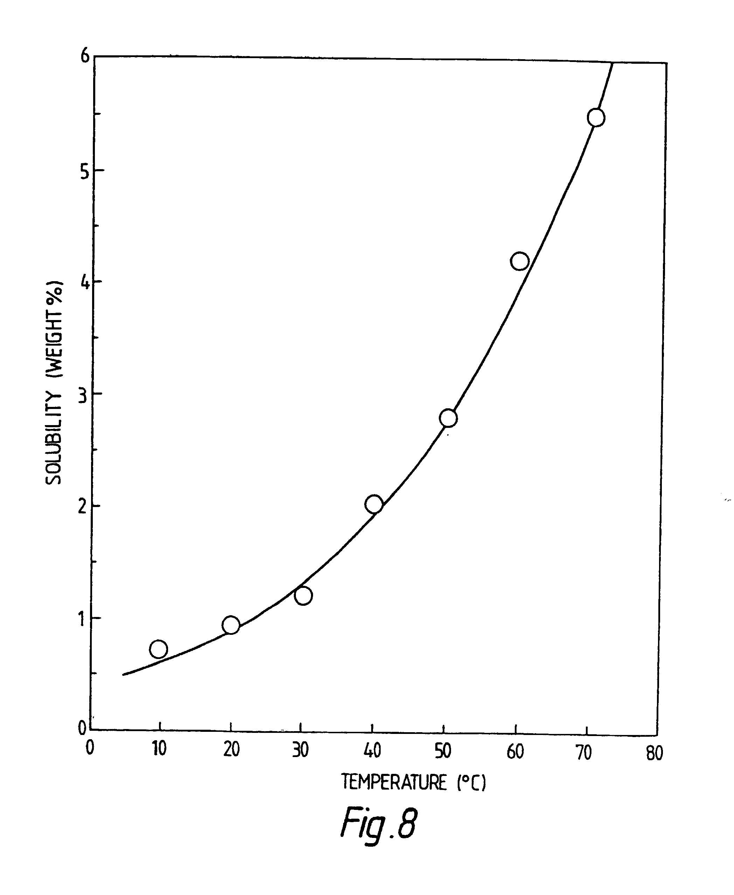

Fig. 8 shows the upper limit of the solubility of APM in water.

The upper limit of the concentration depends upon the stability of APM in solution at elevated temperatures and its soluble concentration and, in the aqueous solution a concentration of about 10% or less, which is a saturation concentration of APM at 80°C, is a usually suitable upper limit.

As the crystallizing solvent, water suffices, which water may optionally contain other solvents as long as one does not deviate from the spirit of the present invention, i.e. no particular difficulty is encountered in the practice of the present invention.

For effectively practicing the process of the present invention, the cooling rate is an important procedure factor. However, in the cooling step based on heat transfer by conduction, a temperature distribution appears within the solution being cooled, and the cooling rate is not constant with time, and thus a definite control of the cooling rate is difficult. However, the average temperature of a cooled solution after a given period of time is determined by the temperature of the solution to be cooled, and the maximum distance between cooled solution and the heat-transfer surface. The initial temperature of the solution depends upon the aforesaid concentration of APM and, as a refrigeration medium, a known medium such a propylene glycol, ethylene glycol or cooling water may be used. The temperature of the refrigeration medium is most suitably - 5° C to 35° C in view of the prevention of freezing of the solvent, and the time required for cooling. Further, as to the maximum distance between the solution and the heat-transfer surface, the larger the distance, the more the difference in crystallization degree due to greater temperature distribution within the cooled solution. In addition, the decomposition of APM proceeds so much that predetermined supersaturation cannot be attained, the separability thus being adversely affected. Therefore, even the remotest part of the solution being cooled is desirably 500 mm or less from the heat-transfer surface. In any case. those skilled in the art can easily find conditions necessary for rendering the whole solution into a pseudo solid phase in the illustrated crystallizers, through simple preliminary experiments.

The thus obtained sherbet-like pseudo solid phase comprising APM crystals, and the solvent does not itself have any fluidity, but has extremely good separating properties from the cooling surface, thus causing no difficulty upon discharge from the crystallizer. It can be easily converted into a slurry, for example, by stirring and can be transported through pumps or the like.

Additionally, in the process of the present invention, the cooling of the system is conducted through heat transfer by conduction, and hence it requires a longer time to cool to a desired temperature than in cooling under forced flow. Needless to say, the process of the present invention provides more advantages than compensate for the disadvantage. However, in order to more raise the efficiency and improve the yield, it is possible to conduct a desupersaturating procedure subsequent to the aforesaid crystallizing step.

Thus, the sherbet-like pseudo solid phase obtained by crystallization through heat transfer by conduction and comprising APM crystals and the solvent are rapidly cooled subsequent to destruction of the solid phase, for example, by stirring to thereby remove the residual supersaturation in a short time. However, where the proportion of APM crystals additionally precipitated in the desupersaturating procedure accounts for about 25% or more of the whole solid phase APM finally obtained, the solid-liquid separability of the slurry sharply deteriorates. Therefore, the desupersaturation is desirably controlled to less than the above-described degree.

The present invention will now be illustrated by the following Examples.

### Example 1

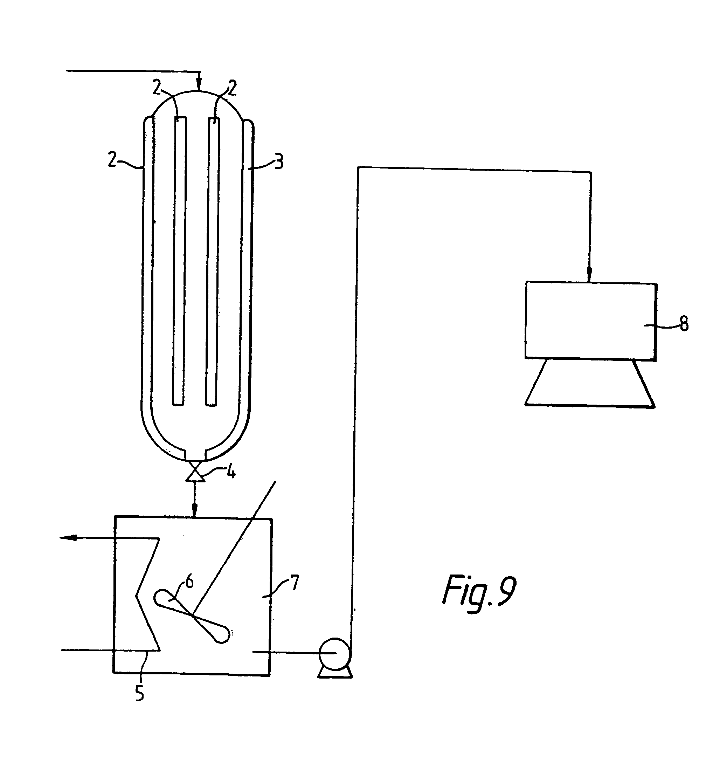

This example was conducted using an apparatus shown in Fig. 9.

380 Litres of a feed solution having dissolved therein 17.7 kg of APM containing 3% of DKP (temperature=55°C; initial concentration of APM=4.4 wt %) was charged in a stainless steel crystallizer having a diameter of 400 mm (maximum distance between the cooled solution and the cooling surface=75 mm) and having jacket 3 and inner cooling plates 2. A refrigeration medium at 0° C was circulated through the jacket and the cooling plates to conduct cooling for 3 hours, during which cooling through heat transfer by conduction became predominant about 15 minutes after initiation of the cooling. The whole solution became a pseudo solid phase after about one hour.

Thereafter, the contents were discharged into tank 7 equipped with cooling coil 5 and stirrer 6 to destroy the solid phase. On this occasion, the average temperature of the slurry was about 16° C, and the APM concentration of the mother liquor was 0.9 wt %. Then, a refrigeration medium was introduced into the coil under further stirring to conduct cooling for one hour to thereby lower the temperature of the slurry to about 7° C. The APM concentration of the mother liquor was 0.7 wt %.

When the thus obtained slurry was filtered and dewatered in a centrifugal separator 8 having a diameter of 36 inches (91.4 cm), the water content of the cake decreased to only 25% after 20 minutes. The yield was 19 kg (wet), the recovery ratio was 86%, and the D KP content was 0.1%.

Furthermore, APM crystals additionally precipitated in the desupersaturation procedure accounted for about 5% of the whole solid phase finally obtained.

Similar results were obtained by crystallizing APM using an apparatus having a cooling tube in place of the cooling plate.

In the case of a slurry obtained by a conventional process (see the Comparative Example below), the water content was as high as 45 to 50% even after 2 hours of filtration and 1 hour of dewatering (3 hours altogether).

### Comparative Example

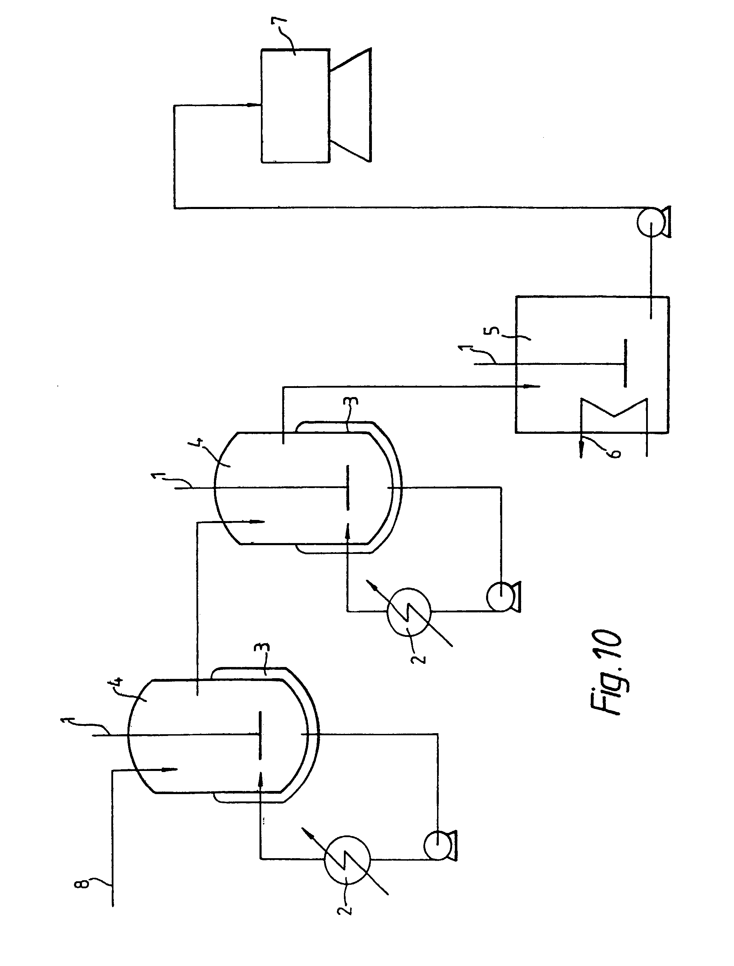

This comparative example was conducted using an apparatus shown in Fig. 10. A feed solution was continuously introduced through feed inlet 8. Two stainless steel tanks 4 (volume = 100 litres) equipped with stirrer 1, outer heat-exchanger 2, and jacket 3 were used in series. The stirring speed was 50 r.p.m., the APM concentration of the feed solution was 4.4 wt %, and the flow rate was 60 litres/hr. The average temperature in the first tank was 25° C, and that in the second tank 10° C. Additionally, there are used a receiving tank 5 equipped with a stirrer 1, and cooling coil 6, and a centrifugal separator 7.

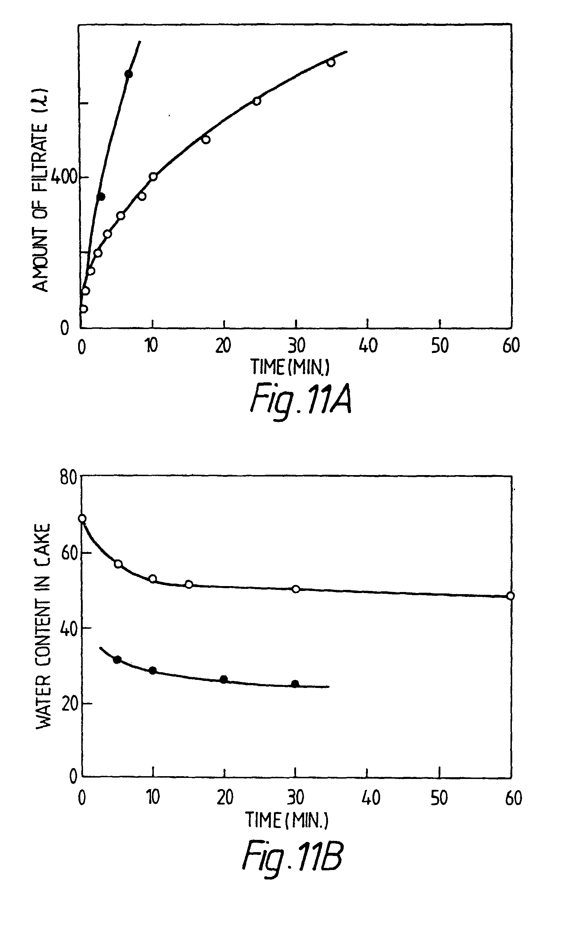

The results of comparing the process of the present invention with the conventional process with respect to the centrifuge filtration rate and the centrifuge dewatering rate are shown in Fig. 11A and Fig. 11B, respectively, wherein 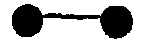 shows the measured values in the case of an APM slurry in accordance with the present invention, and 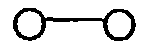 shows the measured values in the case of an APM slurry obtained by the conventional process.

In leaf test by suction filtration to determine specific resistance value, the APM slurry obtained by the process of the present invention had a specific resistance of 1 × 10⁸ to 2 × 10⁸ m/kg immediately after being discharged and 3 × 10⁸ to 5 × 10⁸ m/kg after desupersaturation, whereas the slurry obtained by the conventional process had a specific resistance of 5 × 10¹⁰ to 1 × 10¹¹ m/kg.

### Example 2

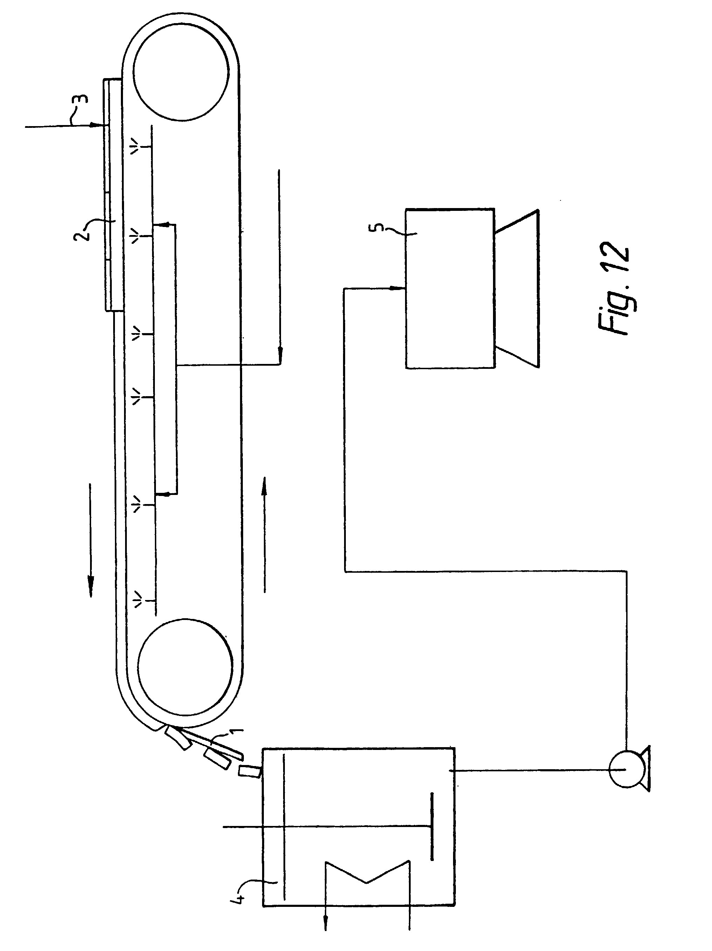

A feed solution having the same composition as in Example 1 was cooled using a stainless steel belt cooler (1.2 m × 5 m) as shown in Fig. 12 to crystallize APM. The feed solution was continuously introduced onto the belt through feed inlet 3. Where feed amount is large, it is preferable to provide guides 2 on the sides of the belt for preventing overflow. In such cases, the guides are not necessarily provided over the full length of the belt, because the solution does not flow out after being rendered sherbet-like.

Cooling was conducted indirectly by forcing a jet of cooling water at 12° C onto the back of the belt. The solution-feeding rate and the belt speed were adjusted so that the thickness of the sherbet, or the maximum distance from the cooling surface, was about 10 mm.

The thus obtained sherbet-containing APM crystals and water was scraped out by scraper 1 and converted in receiving tank 4 by stirring (60 r.p.m.) into a slurry. The average temperature of the product immediately after scraping was about 18° C. Additionally, cooling for desupersaturation was not particularly conducted in the receiving tank.

When about 1 00 litres of the slurry in the receiving tank was subjected to solid-liquid separation in centrifugal separator 5, the water content of cake was reduced to about 30% after 30 minutes. The yield was 4.3 kg. The separated mother liquor contained about 1.5 wt % APM, and the recovery ratio was 68%.

The steel-belt cooler system has the advantages that, as compared to the system used in Example 1, cooling surface can be smaller due to the larger processing speed, and that, in view of the process flow, the feed solution need not necessarily be kept at a high temperature because of the use of a continuous system, the decomposition of APM thus being remarkably reduced.

As is clear from the above, the application of the process of the present invention to the crystallization and separation of APM crystals has the following outstanding industrial advantages as compared to conventional processes (such as, for example, the use of a forced circulation-outer cooling system or a self evaporation system) even though the energy required for cooling, etc., is almost the same:
(1) As to the solid-liquid separation of a slurry containing APM crystals, in the case of a slurry obtained by the conventional process, it is difficult to reduce its water content to the degree attained by a slurry obtained in the process of the present invention, even when separation time is prolonged;
(2) In repeatedly conducting the above-described separation procedure, a slurry obtained by the conventional process is subject to extensive press solidification of the base layer of the cake and its removal requires much labour, whereas a slurry obtained by the process of the present invention does not undergo such phenomenon. For example, in one embodiment of the process of the present invention, the base layer could be easily removed from the filter even after 20 repetitions of the procedure, whereas repeated procedure according to the conventional process resulted in extensive solidification after only 5 repetitions of the procedure, the thus solidified base layer being difficult to remove;
(3) The filtration time and the separation load such as required for cake removal, is, in the process of the present invention, in terms of the required filter area, reduced to about one tenth or less, than in the case of the conventional process;
(4) Additiorrally, the remarkable improvement in separability is accompanied by a reduction in the adhesion of impurity-containing mother liquor on the crystals to one half, and an improvement in the washing effect, and hence the crude crystallization step may be eliminated by combining the cake washing procedure or the like;
(5) The load in the drying step is decreased to about one third. For example the drying load necessary for obtaining 100 kg of dry product powder (water content=3%) is as follows. With APM crystals containing 50% of water and obtained by the conventional process, the load is 5.1 × 10⁴Kcal (neglecting loss in heat transfer procedures) whereas with APM crystals containing 25% water and obtained by the process of the present invention, it is 1.6 × 10⁴Kcal;
(6) The properties of the dry powder are improved, as shown in Table 1, so that the handling properties of the powder are improved.

## Claims

1. A process for crystallizing L-α-aspartyl-L-phenylalanine methyl ester on an industrial scale from its aqueous solution by cooling in an industrial crystallizer, which comprises adjusting the initial concentration of the ester so that the amount of precipitated solid phase formed after cooling is about 10 g or more per litre of solvent, cooling the solution by conductive heat transfer to form an apparently sherbet-like pseudo solid phase without effecting forced flow (that is to say without mechanical stirring or the like), and, if necessary, further cooling the system after formation of the pseudo solid phase, converting said pseudo solid phase to a slurry, subjecting the slurry to a solid-liquid separation, and drying the crystals of L-α-aspartyl-L-phenylalanine methyl ester; wherein said sherbet-like pseudo solid phase comprises bundle-like crystal aggregates of L-α-aspartyl-L-phenylalanine methyl ester and the solvent, has no fluidity, and may be converted into a slurry by stirring.

2. A process as claimed in claim 1, wherein the initial concentration of the L-α-aspartyl-L-phenylalanine methyl ester in the aqueous system is from 2 to 10 wt%.

3. A process as claimed in claim 1, wherein the initial concentration of the L-α-aspartyl-L-phenylalanine methyl ester in the aqueous system is from 3 to 10 wt%.

4. A process as claimed in claim 1, 2 or 3, wherein the temperature of the refrigeration medium used in the process is from -5° to 35°C.

5. A process as claimed in any of claims 1 to 4, wherein the maximum distance between the cooled solution and the cooling surface is 500 mm or less.

6. A process as claimed in any of claims 1 to 5, wherein desupersaturation is carried out by cooling and/or by effecting forced flow, after the formation of the pseudo solid phase.

7. A process as claimed in claim 6, wherein the proportion of L-α-phenylalanine methyl ester crystals additionally precipitated in the desupersaturation procedure is about 25% or less based on the total solid phase finally obtained.

## Patentansprüche

1. Verfahren zum Kristallisieren von L-α-Aspartyl-L-phenylalanin-methylester im industriellen Maßstab aus dessen wäßriger Lösung durch Kühlen in einer industriellen Kristallisationsvorrichtung, welches das Einstellen der Anfangskonzentration des Esters derart, daß die Menge der nach dem Kühlen gebildeten abgeschiedenen festen Phase etwa 10 g oder mehr pro Liter des Lösungsmittels beträgt, das Kühlen der Lösung durch Wärmeleitung ohne Erzeugen einer Zwangsströmung (d.h. ohne mechanisches Rühren oder dergleichen) unter Bildung einer scheinbar sorbetartigen Pseudofestphase, und, falls erforderlich, weiteres Kühlen des Systems nach der Bildung der Pseudofestphase, das Umwandeln dieser Pseudofestphase in eine Aufschlämmung, Behandeln der Aufschlämmung durch Fest-Flüssigphasen-Trennung und Trocknen der Kristalle des L-α-Aspartyl-L-phenylalanin-methylesters umfaßt, wobei die sorbetartige Pseudofestphase bündelartige Kristallaggregate von L-α-Aspartyl-l-phenylalanin-methylester und das Lösungsmittel enthält, keine Fluidität besitzt und durch Rühren in eine Aufschlämmung umgewandelt werden kann.

2. Verfahren nach Anspruch 1, wobei die Anfangskonzentration des L-α-Aspartyl-L-phenylalanin-methylesters in dem wäßrigen System 2 bis 10 Gew.-% beträgt.

3. Verfahren nach Anspruch 1, wobei die Anfangskonzentration des L-α-Aspartyl-L-phenylalanin-methylesters in dem wäßrigen System 3 bis 10 Gew.-% beträgt.

4. Verfahren nach Anspruch 1, 2 oder 3, wobei die Temperatur des bei dem Verfahren verwendeten Kühlmediums -5°C bis 35°C beträgt.

5. Verfahren nach einem der Ansprüche 1 bis 4, wobei der größte Abstand zwischen der gekühlten Lösung und der Kühlfläche 500 mm oder weniger beträgt.

6. Verfahren nach einem der Ansprüche 1 bis 5, wobei das Brechen der Übersättigung durch Kühlen und/oder durch Zwangsströmung nach der Bildung der Pseudofestphase erfolgt.

7. Verfahren nach Anspruch 6, wobei der Anteil an L-α-Phenylalaninmethylester-Kristallen, die zusätzlich bei dem Verfahren des Brechens der Übersättigung ausgefällt werden, etwa 25 % oder weniger, bezogen auf die endgültig erhaltene Gesamtmenge der Festphase beträgt.

## Revendications

1. Procédé pour la cristallisation de l'ester méthylique de la L-α-aspartyl-L-phénylalanine à une échelle industrielle à partir de sa solution aqueuse par refroidissement dans une unité de cristallisation industrielle, qui comprend l'ajustement de la concentration initiale de l'ester pour que la quantité de phase solide précipitée formée après refroidissement soit d'environ 10 g ou plus par litre de solvant, le refroidissement de la solution par transfert de chaleur par conduction pour former une phase pseudo-solide apparemment semblable à un sorbet sans effectuer d'écoulement forcé (c'est-à-dire sans agitation mécanique ou similaires) et, au besoin, le refroidissement complémentaire du système après formation de la phase pseudo-solide, la conversion de ladite phase pseudo-solide en une suspension, la soumission de la suspension à une séparation solide-liquide et le séchage des cristaux d'ester méthylique de la L-α-aspartyl-L-phénylalanine; où ladite phase pseudo-solide semblable à un sorbet comprend des agrégats de cristaux en faisceau d'ester méthylique de la L-α-aspartyl-L-phénylalanine et le solvant, n'a pas de fluidité et peut être convertie en une suspension par agitation.

2. Procédé selon la revendication 1, où la concentration initiale de l'ester méthylique de la L-α-aspartyl-L-phénylalanine dans le système aqueux est de 2 à 10 % en poids.

3. Procédé selon la revendication 1, où la concentration initiale de l'ester méthylique de la L-α-aspartyl-L-phénylalanine dans le système aqueux est de 3 à 10 % en poids.

4. Procédé selon la revendication 1, 2 ou 3, où la température de l'agent de réfrigération utilisé dans le procédé est de -5 à 35°C.

5. Procédé selon l'une quclconque des revendications 1 à 4, où la distance maximale entre la solution refroidie et la surface de refroidissement est de 500 mm ou moins.

6. Procédé selon l'une quelconque des revendications 1 à 5, où une désursaturation est effectuée par refroidissement et/ou écoulement forcé, après la formation de la phase pseudo-solide.

7. Procédé selon la revendication 6, où la proportion de cristaux d'ester méthylique de la L-α-aspartyl-L-phénylalanine précipités en plus dans l'opération de désursaturation est d'environ 25 % ou moins par rapport à la phase solide totale obtenue finalement.
